# EUROPÄISCHE PATENTSCHRIFT

(11) **EP 3 490 972 B1**
(45) Veröffentlichungstag und Bekanntmachung des Hinweises auf die Patenterteilung: **23.09.2020**
(21) Anmeldenummer: 17742239.1
(22) Anmeldetag: 20.07.2017
(51) Int. Cl.: C07C 253/20

(54) **VERFAHREN ZUR HERSTELLUNG VON FLUORALKYLNITRILEN UND DEN ENTSPRECHENDEN FLUORALKYLTETRAZOLEN**
VERFAHREN ZUR HERSTELLUNG VON FLUORALKYLNITRILEN UND DEN ENTSPRECHENDEN FLUORALKYLTETRAZOLEN
PROCÉDÉ DE PRODUCTION DE FLUOROALKYLNITRILES ET DES FLUOROALKYLTÉTRAZOLES CORRESPONDANTS

(30) Priorität: 28.07.2016 EP 16181723
(43) Veröffentlichungstag der Anmeldung: 05.06.2019
(73) Patentinhaber: Bayer CropScience Aktiengesellschaft, 40789 Monheim am Rhein (DE)
(72) Erfinder: HÄSELHOFF, Claus-Christian, 45968 Gladbeck (DE); SCHNATTERER, Albert, 51373 Leverkusen (DE); VERMEHREN, Jan, 65510 Idstein (DE)
(74) Vertreter: BIP Patents
(86) Internationale Anmeldenummer: PCT/EP2017/068328
(87) Internationale Veröffentlichungsnummer: WO 2018/019693

(56) Entgegenhaltungen:
- WO-A1-2010/142377
- DE-T2- 69 428 783
- US-A- 2 730 543

## Beschreibung

Die vorliegende Erfindung betrifft ein Verfahren zur Herstellung von Fluoralkylnitrilen und den entsprechenden Fluoralkyltetrazolen ausgehend von Fluoralkylcarbonsäureamiden.

Fluoralkylnitrile und die entsprechenden Fluoralkyltetrazole sind wichtige Zwischenprodukte zur Herstellung von agrochemischen Wirkstoffen.

Grunewald et al. (J. Med. Chem. 2006, 49, 2939-2952) und auch Swarts (Bulletin des Societes Chimiques Belges, 1922, Vol 31, 364- 365) beschreiben die Herstellung von Difluoracetonitril ausgehend von Difluoracetamid mit Phosphorpentoxid. Hierbei werden die beiden Feststoffe erhitzt und das leichtflüchtige Nitril bei -78°C kondensiert. Der im Reaktionsgefäß zurückbleibende feste Reaktionsrückstand ist jedoch schwer zu entfernen.

Parker in Synthetic Communications (Volume 34, 2004, Pages 903 - 907) und EP 729940A2 beschreiben die Herstellung von fluorierten Nitrilen durch Umsetzung der entsprechenden Amide mit Trifluoressigsäureanhydrid in Pyridin. Nachteilig bei diesem Prozess ist der Einsatz von teurem Trifluoressigsäureanhydrid, das stöchiometrisch eingesetzt werden muss.

CN 102746190A (2012) beschreibt die Herstellung von Trifluoracetonitril aus dem Amid durch das Katalysatorsystem Polyphosphorsäure/Phosphorsäure.

CN 103804231A (2014) offenbart die Herstellung von Trifluoracetonitril aus dem Amid durch Zugabe von Trifluoressigsäureanhydrid in Kohlenstofftetrachlorid.

WO 2010/142377A1 beschreibt die Herstellung von Fluoralkylnitrilen durch Umsetzung von fluorierten Carbonsäureamiden mit Säurehalogeniden und fluorierten Carbonsäuren.

Ein Problem bei der Verwendung von Säurehalogeniden als Dehydratisierungsmittel ist, dass Nebenprodukte wie z.B. Halogenwasserstoff entstehen. Da die Fluoralkylnitrile häufig zu den entsprechenden Fluoralkyltetrazolen beispielsweise mit Natriumazid und Acetonitril umgesetzt werden (siehe auch Radies in Journal of Fluorine Chemistry (2008, 129, 1199-1205)), ist die Entstehung der instabilen und äußerst explosiven Stickstoffwasserstoffsäure (HN₃) durch Reaktion des entsprechenden Halogenwasserstoffs mit dem Natriumazid möglich, jedoch eigentlich absolut unerwünscht.

Jones in Journal of Organic Chemistry (1943, 65, 1458) beschreibt die Dehydratisierung von Trifluoracetamid mittels Phosphorpentoxid. Alle oben beschriebenen Verfahren sind gekennzeichnet dadurch, dass man Spezialapparaturen, sehr hohe Temperaturen, teure und gefährliche Reagenzien benötigt; die gewünschten Produkte nur durch aufwendige Isolierung aus den Produktgemischen isolieren kann.

DE 69428783T2 offenbart neben weiteren Entwässerungsmitteln auch Phosphoroxychlorid (POCl₃) als geeignet zur Herstellung von Carbonsäurenitrilen. Fluorierte Derivate werden dort allerdings nicht beschrieben.

Ausgehend von diesem Stand der Technik ergibt sich als Aufgabe der vorliegenden Erfindung, ein möglichst sicheres Verfahren (d.h. z.B. vollständige Vermeidung der Entstehung von HN₃) zur Herstellung von fluorierten Alkylnitrilen bzw. den daraus erhältlichen fluorierten Alkyltetrazolen bereitzustellen, welches vorzugsweise einfach und kostengünstig durchzuführen ist. Die mit diesem angestrebten Verfahren erhältlichen fluorierten Alkylnitrile bzw. fluorierten Alkyltetrazole sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Die Aufgabe wurde gemäß der vorliegenden Erfindung gelöst durch Verfahren zum Herstellen von Fluoralkylnitrilen der allgemeinen Formel (I), 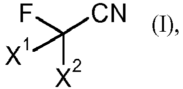 in welcher
X¹ und X² unabhängig voneinander für Fluor, Chlor, Wasserstoff, Methyl stehen,
   dadurch gekennzeichnet, dass man
   fluorierte Carbonsäureamide der Formel (II) 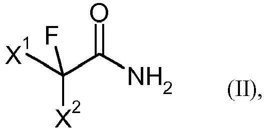 in welcher X¹ und X² die oben genannten Bedeutungen haben,
   in Gegenwart einer Base mit Phosphortrichlorid (PCl₃) und/oder Phosphoroxychlorid (POCl₃) umsetzt.
   X¹ und X² stehen unabhängig voneinander bevorzugt für Fluor.

In einer bevorzugten Ausführungsform der Erfindung werden die Fluoralkylnitrile der allgemeinen Formel (I), 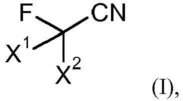 nach Herstellung mit dem oben beschriebenen Verfahren mit Natriumazid in Gegenwart eines Lösungsmittels direkt zu den entsprechenden Fluoralkyltetrazolen der allgemeinen Formel (III) 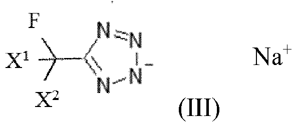 in welcher X¹ und X² die oben genannten Bedeutungen haben,
umgesetzt.

Die Menge an Natriumazid soll für diesen Schritt ausreichend groß sein, damit eine vollständige Umsetzung mit dem Nitril in einer technisch sinnvollen Zeit erfolgen kann. Das molare Verhältnis Natriumazid zu Fluoralkylnitril der allgemeinen Formel (I) liegt bevorzugt zwischen 1 und 10, bevorzugter zwischen 1 und 5 und besonders bevorzugt zwischen 1 und 2. Die Menge an Lösemittel, in der das Azid-Salz gelöst oder suspendiert ist, ist nicht kritisch. Typische Mischungen können bis zu 20 Gew.-% Azid enthalten.

Überraschenderweise lassen sich die fluorierten Alkylnitrile der Formel (I) bzw. daraus entsprechend hergestellten Fluoralkyltetrazole der allgemeinen Formel (III) unter den erfindungsgemäßen Bedingungen sicher und mit guten Ausbeuten in hoher Reinheit herstellen, womit das erfindungsgemäße Verfahren die im Zusammenhang mit dem Stand der Technik beschriebenen Nachteile nicht aufweist.

### Allgemeine Definitionen

Im Zusammenhang mit der vorliegenden Erfindung umfasst der Begriff Halogene bzw. Halogenide, soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

### Phosphortrichlorid (PCl₃) und/oder Phosphoroxychlorid (POCl₃)

Zur Herstellung von Fluoralkylnitrilen der allgemeinen Formel (I) wird u.a. Phosphortrichlorid (PCl₃) und/oder (vorzugsweise oder) Phosphoroxychlorid (POCl₃) eingesetzt.

Das molare Verhältnis von Phosphortrichlorid (PCl₃) bzw. Phosphoroxychlorid (POCl₃) zum eingesetzten fluorierten Alkylamid der allgemeinen Formel (II) kann beispielsweise von 0,05 bis 1, bevorzugt 0,5 bis 0,9, sein. Der Einsatz größerer Mengen (größere molare Verhältnisse als 1) an Phosphortrichlorid (PCl₃) bzw. Phosphoroxychlorid (POCl₃)ist unkritisch aber unwirtschaftlich.

### Base

Das erfindungsgemäße Verfahren zur Herstellung von Fluoralkylnitrilen der allgemeinen Formel (I) wird in Gegenwart einer Base durchgeführt. Als Basen eignen sich beispielsweise Pyridin oder substituierte Pyridine und substituierte oder unsubstituierte Chinoline. Vorzugsweise werden Pyridin oder substituierte Pyridine und substituierte oder unsubstituierte Chinoline eingesetzt. Besonders bevorzugte Beispiele für Basen sind Pyridin, Picoline, Chinolin, Chinaldin und halogenierte Pyridine. Ganz besonders bevorzugt wird 3-Picolin verwendet.

Das molare Verhältnis von Base zum eingesetzten fluorierten Alkylamid der allgemeinen Formel (II) kann beispielsweise von 1 bis 10, besonders bevorzugt 3 bis 6 sein.

Der Einsatz größerer Mengen an Base ist unkritisch aber unwirtschaftlich.

Die Umsetzung zur Herstellung der Verbindungen der allgemeinen Formel (I) bzw. auch der Folgereaktion zu Verbindungen der allgemeinen Formel (III) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (I) bei einer Temperatur von -50 bis 250 °C, vorzugsweise 0 bis 170 °C, durchgeführt werden. Besonders bevorzugt wird die Umsetzung bei Temperaturen von 10 bis 140 °C durchgeführt.

Die gemäß der vorliegenden Erfindung verwendeten fluorierten Alkylamide der Formel (II) sind kommerziell erhältlich oder können nach literaturbekannten Verfahren leicht hergestellt werden (WO 03/080563).

### Lösungsmittel

Die Umsetzung des fluorierten Alkylamids der Formel (II) zur Verbindung mit der Formel (I) kann optional in Gegenwart eines Lösungsmittels durchgeführt werden. Vorzugsweise wird bei der Reaktion auf ein zusätzliches Lösungsmittel verzichtet.

Die Umsetzung des fluorierten Alkylnitrils der Formel (I) zur Verbindung mit der Formel (III) wird in Gegenwart eines Lösungsmittels durchgeführt. Vorzugsweise wird für diese Umsetzung ein aprotischpolares Lösungsmittel wie z.B. ein Keton wie Aceton, ein Lacton wie γ-Butyrolacton, ein Lactam wie N-Methyl-2-pyrrolidon, ein Nitril wie Acetonitril, eine Nitroverbindung wie Nitromethan, ein tertiäres Carbonsäureamid wie Dimethylformamid, ein Harnstoffderivat wie Tetramethylharnstoff oder ein Dimethylpropylenharnstoff (DMPU), ein Sulfoxid wie Dimethylsulfoxid (DMSO), ein Sulfon wie Sulfolan, ein Kohlensäureester wie Dimethylcarbonat oder ein Ethylencarbonat eingesetzt. Besonders bevorzugt wird Aceton oder Acetonitril als Lösungsmittel verwendet.

Die Isolierung der gewünschten Verbindungen der allgemeinen Formel (I) kann beispielsweise durch Destillation erfolgen.

Die Isolierung der gewünschten Verbindungen der allgemeinen Formel (III) kann beispielsweise durch Filtration erfolgen.

Die vorliegende Erfindung wird anhand der nachfolgenden Beispiele näher erläutert, wobei die Beispiele nicht in die Erfindung einschränkende Weise zu interpretieren sind.

### Herstellungsbeispiele

### Beispiel 1

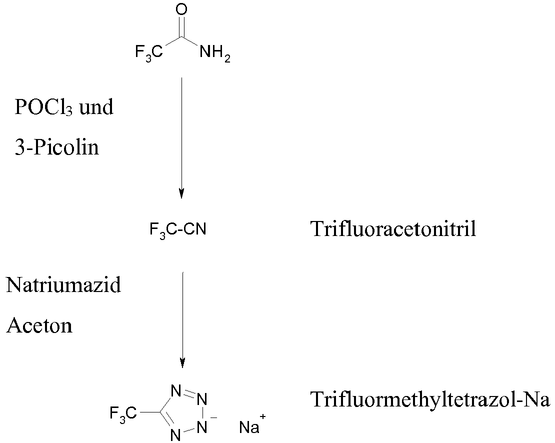

Eine Mischung aus 148,3 g (1,592 Mol, 3,00 Äq.) 3-Picolin und 8,1 g (0,053 Mol; 0,10 Äq.) POCl₃ wurde auf 100°C erhitzt. Zu dieser Mischung wurden 57,0 g (0,372 Mol; 0,70 Äq.) POCl₃ und eine Mischung aus 60,0 g (0,531 Mol; 1,00 Äq.) Trifluoracetamid in 98,9 g (1,062 Mol; 2.00 Äq.) 3-Picolin zeitgleich während einer Zeit von 4 Stunden zu dosiert. Danach wurde die Temperatur während einer Zeitdauer von einer Stunde auf 125°C erhöht. Das entstandene Trifluoracetonitril wird durch einen Rücklaufkondensator und eine Waschflasche, gefüllt mit 3-Picolin, geführt und in eine Mischung aus 36,2 g (0,557 Mol; 1.05 Äq.) Natriumazid und 320,3 g Aceton eingerührt. Die leicht exotherme Reaktion wird bei einer Temperatur von 25 bis 30°C durchgeführt. Am Ende der Umsetzung wird das überschüssige Natriumazid abfiltriert und mit Aceton gewaschen. Das Filtrat wird auf 30% Gewichtsanteile von Trifluormethyltetrazol-Na durch Destillation von Aceton im Vakuum aufkonzentriert. Die theoretische Ausbeute liegt bei 75%.

### Beispiel 2

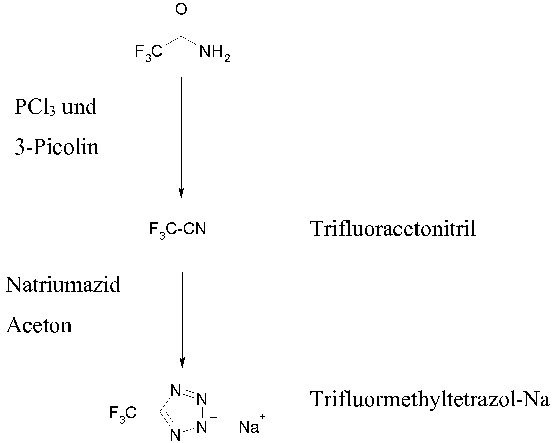

Eine Mischung aus 150,0 g (1,613 Mol; 3,00 Äq.) 3-Picolin und 7,3 g (0,053 Mol; 0,10 Äq.) PCl₃ wurde auf 70°C erhitzt. Zu dieser Mischung wurde 51,0 g (0,372 Mol; 0,70 Äq.) PCl₃ und eine Mischung aus 60,0 g (0,531 Mol; 1,00 Äq.) Trifluoracetamid in 100.0 g (1.074 Mol; 2.00 Äq.) 3-Picolin zeitgleich während einer Zeit von 4 Stunden zudosiert. Danach wurde die Temperatur während einer Zeitdauer von einer Stunde auf 80°C erhöht. Das entstandene Trifluoracetonitril wird durch einen Rücklaufkondensator und eine Waschflasche, gefüllt mit 3-Picolin, geführt und in eine Mischung aus 36,2 g (0,557 Mol; 1.05 Äq.) Natriumazid und 320,0 g Aceton eingerührt. Die leicht exotherme Reaktion wird bei einer Temperatur von 25 bis 30°C durchgeführt. Am Ende der Umsetzung wird das überschüssige Natriumazid abfiltriert und mit Aceton gewaschen. Das Filtrat wird auf 30% Gewichtsanteile von Trifluormethyltetrazol-Na durch Destillation von Aceton im Vakuum aufkonzentriert. Die theoretische Ausbeute liegt bei 63%.

## Patentansprüche

1. Verfahren zum Herstellen von Fluoralkylnitrilen der allgemeinen Formel (I), 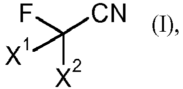 in welcher
X¹ und X² unabhängig voneinander für Fluor, Chlor, Wasserstoff, Methyl stehen,
**dadurch gekennzeichnet, dass** man
fluorierte Carbonsäureamide der Formel (II) 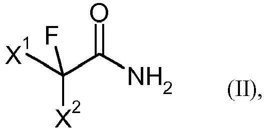 in welcher X¹ und X² die oben genannten Bedeutungen haben,
in Gegenwart einer Base mit Phosphortrichlorid (PCl₃) und/oder Phosphoroxychlorid (POCl₃) umsetzt.

2. Verfahren gemäß Anspruch 1, wobei X¹ und X² jeweils für Fluor stehen.

3. Verfahren gemäß einem der Ansprüche 1 bis 2, wobei das molare Verhältnis von Phosphorhalogenid zum eingesetzten fluorierten Alkylamid der allgemeinen Formel (II) 0,05 bis 1 ist.

4. Verfahren gemäß einem der Ansprüche 1 bis 3, wobei die Base ausgewählt ist aus der Gruppe bestehend aus Pyridin, Picoline, Chinolin, Chinaldin und halogenierte Pyridine.

5. Verfahren gemäß einem der Ansprüche 1 bis 4, wobei das molare Verhältnis von Base zum eingesetzten fluorierten Alkylamid der allgemeinen Formel (II) 1 bis 10 ist.

6. Verfahren gemäß einem der Ansprüche 1 bis 5, **dadurch gekennzeichnet, dass** man die erhaltenen Fluoralkylnitrile der allgemeinen Formel (I), 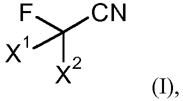 in Gegenwart eines Lösungsmittels mit Natriumazid zu den entsprechenden Fluoralkyltetrazolen der allgemeinen Formel (III) 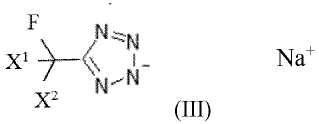 in welcher X¹ und X² die oben genannten Bedeutungen haben, umsetzt.

## Claims

1. Method for preparing fluoroalkylnitriles of the general formula (I), 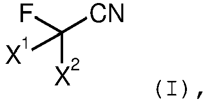 in which
X¹ and X² are mutually independently fluorine, chlorine, hydrogen or methyl,
**characterized in that**
fluorinated carboxamides of the formula (II) 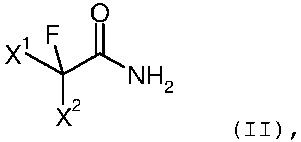 in which X¹ and X² are as defined above,
are reacted with phosphorus trichloride (PCl₃) and/or phosphorus oxychloride (POCl₃) in the presence of a base.

2. Method according to Claim 1, wherein X¹ and X² are both fluorine.

3. Method according to either of Claims 1 and 2, wherein the molar ratio of phosphorus halide to the fluorinated alkylamide used of the general formula (II) is 0.05 to 1.

4. Method according to any of Claims 1 to 3, wherein the base is selected from the group consisting of pyridine, picolines, quinoline, quinaldine and halogenated pyridines.

5. Method according to any of Claims 1 to 4, wherein the molar ratio of base to the fluorinated alkylamide used of the general formula (II) is 1 to 10.

6. Method according to any of Claims 1 to 5, **characterized in that** the fluoroalkylnitriles obtained of the general formula (I), 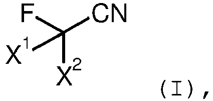 are reacted with sodium azide in the presence of a solvent to give the corresponding fluoroalkyltetrazoles of the general formula (III) 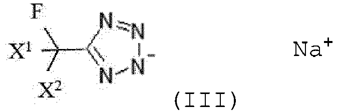 in which X¹ and X² are as defined above.

## Revendications

1. Procédé pour la préparation de fluoroalkylnitriles de formule générale (I), 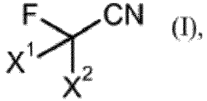 dans laquelle
X¹ et X² représentent indépendamment l'un de l'autre fluor, chlore, hydrogène, méthyle,
**caractérisé en ce qu'**on transforme
des amides d'acides carboxyliques fluorés de formule (II) 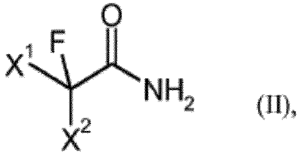 dans laquelle X¹ et X² possèdent les significations mentionnées ci-dessus,
en présence d'une base avec du trichlorure de phosphore (PCl₃) et/ou de l'oxychlorure de phosphore (POCl₃).

2. Procédé selon la revendication 1, X¹ et X² représentant à chaque fois fluor.

3. Procédé selon l'une quelconque des revendications 1 et 2, le rapport molaire d'halogénure de phosphore sur l'alkylamide fluoré utilisé de formule générale (II) étant de 0,05 à 1.

4. Procédé selon l'une quelconque des revendications 1 à 3, la base étant choisie dans le groupe constitué par la pyridine, la picoline, la quinoléine, la quinaldine et les pyridines halogénées.

5. Procédé selon l'une quelconque des revendications 1 à 4, le rapport molaire de la base sur l'alkylamide fluoré utilisé de formule générale (II) étant de 1 à 10.

6. Procédé selon l'une quelconque des revendications 1 à 5, **caractérisé en ce qu'**on transforme les fluoroalkylnitriles obtenus de formule générale (I), 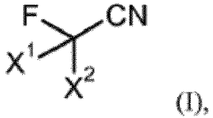 en présence d'un solvant avec de l'azoture de sodium en fluoroalkyltétrazoles correspondants de formule générale (III) 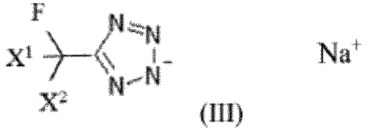 dans laquelle X¹ et X² possèdent les significations mentionnées ci-dessus.
